# FASCICULE DE BREVET EUROPEEN

(11) **EP 1 989 310 B1**
(45) Date de publication et mention de la délivrance du brevet: **04.11.2015**
(21) Numéro de dépôt: 07731065.4
(22) Date de dépôt: 28.02.2007
(51) Int. Cl.: C12N 15/53, C12N 15/81, C12N 9/04, C12N 1/16, C12P 7/22, C12P 7/24, C12P 7/42

(54) **SYSTÈME D'EXPRESSION CHEZ LA LEVURE POUR LA PRODUCTION DE MOLÉCULES AROMATIQUES**
SYSTEM ZUR HERSTELLUNG AROMATISCHER MOLEKÜLE MITTELS BIOKONVERSION
SYSTEM FOR PRODUCING AROMATIC MOLECULES BY BIOCONVERSION

(30) Priorité: 01.03.2006 FR 0601838
(43) Date de publication de la demande: 12.11.2008
(73) Titulaire: V. Mane Fils, 06620 Bar sur Loup (FR)
(72) Inventeur: ZUCCA, Joseph, 06130 Grasse (FR); LAMBERT, Fanny, 06580 Pegomas (FR); MANE, Jean, F-06130 Grasse (FR); NESS, Frédéreque, 33000 Bordeaux (FR); AIGLE, Michel, 69100 Villeurbanne (FR)
(74) Mandataire: Cabinet Plasseraud
(86) Numéro de dépôt international: PCT/FR2007/000364
(87) Numéro de publication internationale: WO 2007/099230

(56) Documents cités:
- WO-A-01/55342
- WO-A-96/08576
- OVERHAGE JOERG ET AL: "Highly efficient biotransformation of eugenol to ferulic acid and further conversion to vanillin in recombinant strains of Escherichia coli." APPLIED AND ENVIRONMENTAL MICROBIOLOGY, vol. 69, no. 11, novembre 2003 (2003-11), pages 6569-6576, XP002410833 ISSN: 0099-2240 cité dans la demande
- BENEN JACQUES A E ET AL: "Molecular cloning, sequencing, and heterologous expression of the vaoA gene from Penicillium simplicissimum CBS 170.90 encoding vanillyl-alcohol oxidase" JOURNAL OF BIOLOGICAL CHEMISTRY, vol. 273, no. 14, 3 avril 1998 (1998-04-03), pages 7865-7872, XP002410834 ISSN: 0021-9258
- GUELDENER U ET AL: "A new efficient gene disruption cassette for repeated use in budding yeast" NUCLEIC ACIDS RESEARCH, OXFORD UNIVERSITY PRESS, SURREY, GB, vol. 24, no. 13, 1996, pages 2519-2524, XP002147414 ISSN: 0305-1048 cité dans la demande
- BONNEAUD N ET AL: "A FAMILY OF LOW AND HIGH COPY REPLICATIVE INTEGRATIVE AND SINGLE-STRANDED SACCHAROMYCES-CEREVISIAE AND ESCHERICHIA-COLI SHUTTLE VECTORS" YEAST, vol. 7, no. 6, 1991, pages 609-616, XP002410835 ISSN: 0749-503X
- "Rapid Translation System RTS pIVEX His-tag vector Set Cat. No. 3 253 538" ROCHE MOLECULAR BIOMEDICALS, mai 2001 (2001-05), pages 1-6, XP002410836 Roche Diagnostics GmbH roche molecular biomedicals Mannhein Germany
- OVERHAGE JOERG ET AL: "Biotransformation of eugenol to ferulic acid by a recombinant strain of Ralstonia eutropha H16" APPLIED AND ENVIRONMENTAL MICROBIOLOGY, vol. 68, no. 9, septembre 2002 (2002-09), pages 4315-4321, XP002469536 ISSN: 0099-2240
- OVERHAGE J ET AL: "Biotransformation of eugenol to vanillin by a mutant of Pseudomonas sp. strain HR199 constructed by disruption of the vanillin dehydrogenase (vdh) gene" APPLIED MICROBIOLOGY AND BIOTECHNOLOGY, vol. 52, no. 6, novembre 1999 (1999-11), pages 820-828, XP002469537 ISSN: 0175-7598

## Description

La présente invention concerne la production de molécules aromatiques naturelles. Plus particulièrement, l'invention concerne un nouveau système d'expression chez la levure utilisant une cassette d'expression et permettant la production de dérivés phénoliques par bioconversion, lesdits dérivés phénoliques étant utilisables pour la production de molécules aromatiques naturelles employées en aromatique alimentaire ou en parfumerie (arômes ou fragrances).

La production de molécules aromatiques naturelles peut être obtenue soit par voie biologique soit par synthèse chimique. Par exemple, la vanilline peut être obtenue par l'une ou l'autre de ces deux voies.
La vanilline (3-methoxy-4-hydroxybenzaldehyde) est le composant principal responsable des propriétés olfactives et gustatives de l'extrait de vanille dérivé de gousses de *Vanillia planifolia.* Il s'agit de l'une des molécules aromatiques les plus utilisées dans l'industrie. Cependant, la production de vanilline naturelle à partir de gousse de vanille ou d'extrait de vanille ne couvre que 20% de ce marché ; son utilisation est limitée en raison d'une part du potentiel de gousses disponibles au niveau mondial et d'autre part en raison du prix élevé, très fluctuant, de ces gousses (de l'ordre de 30 €/kg à 450 €/kg soit au minimum 1500 €/kg de potentiel en vanilline naturelle).

La vanilline de synthèse est donc fréquemment utilisée comme un substituant bon marché (environ 15 €/kg) de la vanilline naturelle. Cependant, si la vanilline de synthèse convient à des applications en parfumerie et en cosmétique, elle peut soulever des difficultés d'ordre réglementaire dans les industries agro-alimentaires. De plus, les arômes de synthèse sont généralement moins appréciés par les consommateurs que les arômes naturels.

C'est pourquoi l'on cherche à obtenir des molécules aromatiques naturelles, en particulier la vanilline, par des procédés biologiques, notamment de bioconversion, qui mettent en oeuvre des microorganismes (bactéries, levures, moisissures), des cellules végétales ou leurs systèmes enzymatiques.

Au sens de la présente invention, on entend par bioconversion la transformation biologique d'un substrat, de préférence issu d'une source naturelle, pour obtenir des arômes, des fragrances ou des précurseurs d'arômes ou de fragrances naturels.

La vanilline peut être produite suivant le schéma réactionnel suivant :

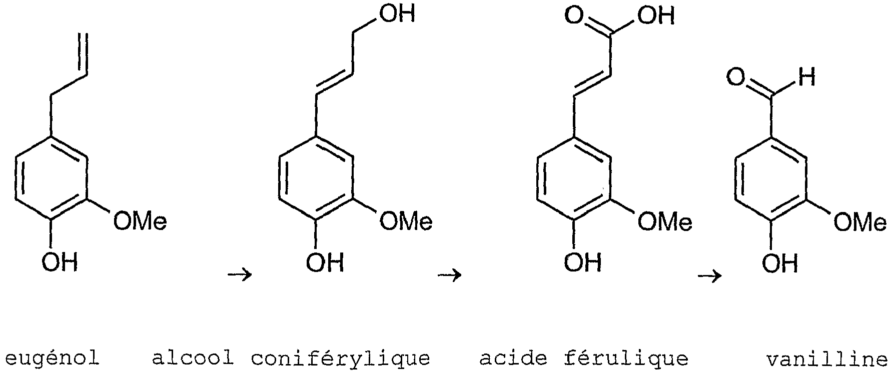

Chacune des molécules citées peut permettre l'accès à la vanilline pour peu qu'elles soient disponibles, sachant que les plus importantes sont l'acide férulique et l'eugénol.

Par exemple, les demandes EP 453 368, PCT WO/96/08576 et PCT WO/00/61721 décrivent des procédés permettant la production de vanilline naturelle par bioconversion à partir d'acide férulique en présence de champignons filamenteux. L'acide férulique utilisé dans ces procédés provient de l'extraction de co-produits agricoles contenant des esters d'acide férulique : maïs, riz, betterave ou blé. Mais la faible concentration en acide férulique de ces co-produits et les multiples étapes nécessaires à son extraction-purification font que le rendement d'extraction à partir de ces esters reste assez faible ; ce qui induit un prix élevé pour cette matière première et donc un prix de revient élevé pour la vanilline qui en dérive.

Pour qu'un procédé biologique employant des microorganismes puisse être rentable, il est donc préférable d'utiliser un substrat plus disponible et bon marché.

C'est le cas de l'eugénol qui peut être source d'alcool coniférylique ou d'acide férulique.

L'article publié par J. Overhage et al. décrit que l'expression du gène de la vanillyl alcool oxydase issu de *Penicillium simplicissimum* dans *Escherichia coli* permet de catalyser la conversion de l'eugénol en alcool coniférylique. Pour convertir l'alcool coniférylique en acide férulique, J. Overhage et al expriment ensuite deux autres enzymes issues de *Penicillium simplicissimum* dans *Escherichia coli :* la coniféryl alcool déshydrogénase et la coniféryl aldéhyde déshydrogénase (Highly efficient biotransformation of eugenol to ferulic acid and further conversion to vanillin in recombinant strains of Escherichia coli, 2003, Applied and Environmental microbiology p 6569-6576). Si ce procédé utilise bien un substrat disponible et peu coûteux, l'eugénol, ce système de production d'acide férulique dans E. coli reste cependant difficile à mettre en oeuvre du fait du triple clonage nécessaire pour convertir de l'eugénol en acide férulique.

La présente invention a donc pour objet un procédé de production de précurseurs de vanilline naturelle ou de vanilline naturelle elle-même, à un coût de production moindre que celui de l'art antérieur, et avec un procédé simple à mettre en oeuvre industriellement.

Les solutions proposées par l'invention sont d'utiliser ou de produire des substrats naturels disponibles, de faible coût comme l'eugénol et l'acide férulique. Ce dernier qui est le substrat le plus utilisé pour la synthèse de la vanilline, est souvent d'un prix élevé et d'une qualité difficile à contrôler.

Un autre objet de l'invention est donc un procédé simple et efficace de production d'acide férulique naturel et/ou d'alcool coniférylique naturel, d'un prix industriellement acceptable. Si l'acide férulique peut être utilisé comme précurseur de la vanilline naturelle, l'alcool coniférylique peut être source d'alcool déhydroconiférylique que l'on rencontre dans des parfums rares comme celui de fleurs de tiaré et peut, par oxydation, être source d'acide férulique.

Le moyen essentiel des procédés selon l'invention est une levure transformée par le gène codant la vanillyl alcool oxydase, du type du gène de la vanillyl alcool oxydase (SEQ ID n°1) issu de *Penicillium Simplicissimum* (référence Genbank Y15627), ou toute séquence nucléotidique ayant au moins 70%, de préférence 80%, très préférentiellement 90%, d'identité avec la séquence de SEQ ID n°1.

Ainsi, un objet de l'invention est de proposer la bioconversion de substrats de faible coût, dans une levure comprenant au moins un gène codant la vanillyl alcool oxydase.

La bioconversion d'eugénol par la levure de l'invention permet d'une part la production d'acide férulique et/ou d'alcool coniférylique naturels, sans impureté et de bas prix, et d'autre part la production de vanilline naturelle.

Selon un mode de réalisation de l'invention, la levure comprend au moins un système d'expression qui contient le gène codant la vanillyl alcool oxydase. Avantageusement, ledit système d'expression comprend :
(1) des moyens visant à l'intégration dudit système dans le génome de ladite cellule, comprenant deux séquences nucléotidiques,
(2) des moyens visant à la sélection de ladite cellule ayant intégré ledit système, comprenant un insert de sélection comprenant deux séquences LoxP encadrant un promoteur, un marqueur de sélection du type gène de résistance aux antibiotiques et un terminateur,
(3) une cassette d'expression du gène codant la vanillyl alcool oxydase, comprenant un promoteur permettant l'expression dudit gène, au moins un site de clonage permettant l'intégration dudit gène, et un terminateur.

On entend par « cassette d'expression » une séquence nucléotidique comprenant un promoteur, un site d'insertion pour le clonage du gène d'intérêt ou un site multiple de clonage (MCS pour Multiple Cloning Site) et un terminateur. On entend par « promoteur » une séquence d'ADN nécessaire à l'initiation et au contrôle de la transcription, par « site d'insertion pour le clonage » ou « site multiple de clonage » une séquence d'ADN contenant un ou plusieurs sites de restriction, et par « terminateur » une séquence d'ADN nécessaire à la terminaison de la transcription. On entend par « vecteur » toute séquence d'ADN dans laquelle il est possible d'insérer des fragments d'acide nucléique étranger, les vecteurs permettant d'introduire de l'ADN étranger dans une cellule hôte. Des exemples de vecteurs sont les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus. On entend par « marqueur de sélection » un gène dont l'expression confère aux cellules qui le contiennent une caractéristique permettant de les sélectionner. Il s'agit par exemple d'un gène de résistance aux antibiotiques.

Selon un mode de réalisation préféré de l'invention, le site de clonage comprend les sites de restriction d'enzymes de restriction, NotI, BamHI, MfeI, XhoI, permettant l'insertion de la séquence nucléotidique de la vanillyl alcool oxydase dans la cassette d'expression. Avantageusement, ce site de clonage a pour séquence la séquence SEQ ID n°2 ou toute séquence identique à au moins 70%, de préférence 80%, très préférentiellement 90%, à la séquence SEQ ID n°2.

Selon un mode de réalisation préféré de l'invention, l'encadrement du marqueur de sélection par deux sites LoxP va permettre l'excision de ce marqueur de sélection grâce au système CRE/Lox. L'enzyme CRE est une recombinase qui reconnaît spécifiquement les sites LoxP. La séquence nucléotidique comprise entre ces deux sites est qualifiée d'ADN cible et sera éliminée en présence de l'enzyme CRE. En effet, lorsque l'enzyme CRE se lie aux sites LoxP, elle coupe ces sites en deux et recolle ensemble deux moitiés après que l'ADN cible ait été éliminé. Avantageusement, les séquences LoxP ont pour séquence la séquence SEQ ID n°3 ou toute séquence identique à au moins 70%, de préférence 80%, très préférentiellement 90%, à la séquence SEQ ID n°3.

Selon un mode de réalisation préféré de la présente invention, les séquences permettant l'intégration du système d'expression dans le génome de ladite levure sont multiples et sont choisies dans le groupe comprenant les séquences TY, les séquences télomériques X et Y', les séquences DUP, la séquence □ ou toutes séquences répétées dans le génome de la levure.
Avantageusement, lesdites séquences sont les séquences TY1A et TY1B de *Saccharomyces cerevisiae,* et ont de préférence pour séquence les séquences SEQ ID n°4 et SEQ ID n°5 respectivement ou toutes séquences qui leur sont identiques à au moins 70%, de préférence 80%, très préférentiellement 90%.

Selon un autre mode de réalisation préféré de l'invention, les promoteurs utilisés dans le système d'expression sont des promoteurs forts, c'est-à-dire des promoteurs qui induisent une forte transcription des gènes sous leur contrôle. Des exemples de promoteurs forts sont 1) des promoteurs gouvernant l'expression de gènes dont les protéines sont abondantes dans la levure comme les promoteurs gouvernant l'expression de protéines de la glycolyse ou du métabolisme de l'azote ; 2) des promoteurs dont l'expression est spécifique, par exemple des promoteurs dont l'activité est régulée par la présence de sucre ou d'azote ; 3) des promoteurs dont l'activité est importante au vu des expériences de transcriptome ; ou 4) des promoteurs artificiels conçus pour permettre une forte transcription des gènes qu'ils régulent. Les terminateurs utilisés dans le système d'expression sont choisis pour leur capacité à permettre une bonne stabilité des ARNm. Avantageusement, le terminateur choisi correspond au promoteur fort choisi précédemment.
Avantageusement, le promoteur permettant l'expression du gène d'intérêt dans le système d'expression est le promoteur du gène TDH3 de *Saccharomyces cerevisiae,* et a de préférence pour séquence la séquence SEQ ID n°6 ou toute séquence identique à au moins 70%, de préférence 80%, très préférentiellement 90%, à la séquence SEQ ID n°6. Avantageusement, le terminateur associé est le terminateur du gène CYC1 de *Saccharomyces cerevisiae,* et a de préférence pour séquence la séquence SEQ ID n°7 ou toute séquence identique à au moins 70%, de préférence 80%, très préférentiellement 90%, à la séquence SEQ ID n°7. Avantageusement, le promoteur et le terminateur permettant l'expression du marqueur de sélection sont le promoteur et le terminateur du gène TEF1 de *Ashbya gossypii,* et ont de préférence pour séquence la séquence SEQ ID n°8 et SEQ ID n°9 respectivement ou toutes séquences qui leur sont identiques à au moins 70%, de préférence 80%, très préférentiellement 90%.

Selon un autre mode de réalisation préféré de l'invention, le marqueur de sélection utilisé dans le système d'expression est un gène de résistance aux antibiotiques choisi dans le groupe comprenant les gènes de résistance à la généticine, nourseothricine, phléomycine, zéocine ou tout autre gène de résistance à un antibiotique dominant pour lequel la levure sauvage est sensible.
Avantageusement, le marqueur de sélection utilisé dans le système d'expression est le gène de résistance à la généticine, et a de préférence pour séquence la séquence SEQ ID n°10 ou toute séquence identique à au moins 70%, de préférence 80%, très préférentiellement 90%, à la séquence SEQ ID n°10.

Selon un autre mode de réalisation préféré de l'invention, le système d'expression contenant le gène de la vanillyl alcool oxydase a pour séquence la séquence nucléotidique de SEQ ID n°11 ou toute séquence identique à au moins 70%, de préférence 80%, très préférentiellement 90%, à la séquence SEQ ID n°11.

La présente invention a également pour objet un vecteur comprenant un système d'expression tel que défini précédemment.

Selon un autre mode de réalisation préféré de l'invention, le vecteur comprenant le système d'expression est un plasmide, et avantageusement pUC57.

La présente demande décrit également des bactéries transformées, comprenant au moins un vecteur tel que défini ci-dessus.
Ces bactéries transformées peuvent appartenir à n'importe quelle espèce permettant la réplication du vecteur support choisi. Avantageusement, il s'agit de bactéries du genre E. Coli.

La présente demande décrit aussi un vecteur d'excision du gène de résistance aux antibiotiques, ledit vecteur d'excision comprenant :
(1) des moyens visant à la sélection des cellules le contenant, comprenant le promoteur et le terminateur du gène TEF1 de *Ashbya gossypii* et le gène de résistance à la nourseothricine, et
(2) des moyens visant à l'excision du marqueur de sélection présent dans le système d'expression, comprenant le promoteur du gène GAL1 de *Saccharomyces cerevisiae,* le gène CRE et le terminateur du gène CYC1 de *Saccharomyces cerevisiae.*
Avantageusement, le vecteur d'excision comprend la séquence SEQ ID n°12 ou toute séquence identique à au moins 70%, de préférence 80%, très préférentiellement 90%, à la séquence SEQ ID n°12.

Selon un mode de réalisation préféré de l'invention, le vecteur d'excision décrit ci-dessus est choisi parmi les vecteurs qui sont mal ségrégés pendant les divisions cellulaires, conduisant par cette caractéristique à une perte du vecteur en absence de pression de sélection fréquente, par exemple des vecteurs réplicatifs de type multicopies.
Avantageusement, le vecteur d'excision est le plasmide pFL44s.

Ce vecteur d'excision a pour premier avantage de permettre l'élimination du gène de résistance aux antibiotiques présent dans le génome des levures à la suite de leur transformation avec le système d'expression. Les levures permettant la production de molécules aromatiques selon l'invention ne contiennent donc pas d'ADN non originaire de souches appartenant au même genre ou à la même famille, à l'exception du gène codant la vanillyl alcool oxydase.

De plus, l'élimination du gène de résistance par le vecteur d'excision permet aussi de transformer plusieurs fois une levure, de façon à augmenter le nombre de copies du système d'expression présentes dans le génome et ainsi augmenter la quantité de protéine vanillyl alcool oxydase produite.

L'invention a pour objet des levures de l'espèce Saccharomyces cerevisiae comprenant le système d'expression contenant la vanillyl alcool oxydase, ledit système d'expression ayant pour séquence la séquence SEQ ID n°11 ou toute séquence identique à au moins 70%, de préférence 80%, très préférentiellement 90%, à la séquence SEQ ID n°11.

L'invention a aussi pour objet des levures de l'espèce Saccharomyces cerevisiae telles que décrites précédemment et/ou transformées avec le vecteur d'excision tel que décrit précédemment.

Selon un mode de réalisation préféré de l'invention, les levures appartiennent à l' espèce Saccharomyces cerevisiae capable de réaliser la bioconversion des précurseurs de vanilline (notamment eugénol, acide férulique, alcool coniférylique) en vanilline selon des procédés connus de l'homme de l'art.

Les levures appartiennent au genre Saccharomyces. Très préférentiellement, les levures proviennent d'une souche cerevisiae.

La présente invention a pour objet un procédé de production d'alcool coniférylique et/ou d'acide férulique naturels, comprenant les étapes suivantes :
a) clonage de la séquence nucléotidique du gène codant la vanillyl alcool oxydase dans le système d'expression,
b) transformation de la levure Saccharomyces cerevisiae avec le système d'expression ainsi obtenu,
c) culture de la levure dans les conditions permettant l'expression de la vanillyl alcool oxydase,
d) mise en contact de la levure avec de l'eugénol.
Selon un mode de réalisation préféré de l'invention, la séquence nucléotidique du gène codant la vanillyl alcool oxydase et le vecteur comprenant le système d'expression sont dans un premier temps digérés par une ou plusieurs enzymes de restriction. La séquence du gène codant la vanillyl alcool oxydase est ensuite insérée par simple ligation ou tout autre moyen d'insertion dans le système d'expression porté par le vecteur.
De façon à amplifier le système d'expression, des bactéries sont transformées selon n'importe quel procédé connu avec le produit de ligation (vecteur portant le système d'expression contenant le gène de la vanillyl alcool oxydase). Les bactéries ayant intégré le système d'expression sont sélectionnées grâce au gène de résistance aux antibiotiques présent sur le vecteur portant le système d'expression contenant le gène codant la vanillyl alcool oxydase.
Dans un second temps, le vecteur portant le système d'expression est digéré pour une ou plusieurs enzymes de restriction, de préférence PvuII, coupant de part et d'autre du système d'expression. Le système d'expression est ensuite purifié puis transformé dans les levures appartenant à l'espèce Saccharomyces cerevisiae selon n'importe quel procédé connu. Les levures ayant intégré le système d'expression sont sélectionnées grâce au gène de résistance présent dans le système d'expression.
Enfin, les levures transformées sont cultivées dans les conditions connues de l'homme du métier permettant l'expression de la vanillyl alcool oxydase. Lorsque l'eugénol est ajouté à la culture, la vanillyl alcool oxydase permet de catalyser sa bioconversion en alcool coniférylique et/ou en acide férulique.

L'invention a aussi pour objet un procédé de production d'alcool coniférylique et/ou d'acide férulique naturels, dans lequel le marqueur de sélection présent dans le génome de la levure a été excisé, ledit procédé comprenant après l'étape b) et avant les étapes c) et d), les étapes b1), b2) et b3) suivantes :
b1) transformation de la levure Saccharomyces cerevisiae avec le vecteur d'excision,
b2) culture de la levure dans les conditions permettant l'expression de l'enzyme CRE,
b3) isolement des levures ayant perdu le marqueur de sélection.
Selon un mode de réalisation préféré de l'invention, la séquence nucléotidique du gène codant la vanillyl alcool oxydase est insérée dans le système d'expression, et les levures Saccharomyces cerevisiae sont dans un premier temps transformées avec ce système d'expression comme décrit précédemment. Les levures ayant intégré le système d'expression sont sélectionnées grâce à la présence d'un premier marqueur de sélection, de préférence un gène de résistance aux antibiotiques. Les levures sélectionnées positivement sont ensuite transformées avec le vecteur d'excision selon n'importe quel procédé connu. Les levures ayant intégré le vecteur d'excision sont sélectionnées grâce au gène de résistance à la nourseothricine présent dans le vecteur d'excision.
Les levures, ayant donc intégré le système d'expression et le vecteur d'excision, sont alors cultivées dans les conditions permettant l'expression de l'enzyme CRE. L'enzyme CRE active va permettre d'exciser le premier marqueur de sélection encadré par les séquences loxP dans le système d'expression. Les levures ayant perdu ce marqueur de sélection sont ensuite sélectionnées. Par exemple, les levures ayant perdu ce premier gène de résistance à un antibiotique sont sélectionnées grâce à leur absence de résistance à cet antibiotique.
Les levures obtenues à la suite de cette étape de sélection ne possèdent plus le marqueur de sélection présent dans le système d'expression, mais possèdent encore le gène de résistance à la nourseothricine présent dans le vecteur d'excision. Tel que décrit précédemment, le vecteur d'excision est choisi parmi les vecteurs qui sont mal ségrégés pendant les divisions cellulaires. De part cette caractéristique, le vecteur d'excision est facilement perdu en absence de pression de sélection fréquente. On entend par pression de sélection tout procédé contribuant à sélectionner les cellules. Par exemple, maintenir en culture des levures possédant un gène de résistance à un antibiotique en présence de cet antibiotique correspond à un procédé de pression de sélection. Les levures obtenues à l'étape précédente sont donc cultivées en absence de pression de sélection de façon à perdre le vecteur d'excision, puis sont sélectionnées pour la perte du gène de résistance à la nourseothricine.
Les levures ainsi obtenues ont intégré dans leur génome le système d'expression permettant l'expression de la vanillyl alcool oxydase, tandis que leur génome ne contient plus de gènes de résistance aux antibiotiques. Les levures transformées sont alors cultivées dans les conditions connues de l'homme du métier permettant l'expression de la vanillyl alcool oxydase. Lorsque l'eugénol est ajouté à la culture, la vanillyl alcool oxydase permet de catalyser sa bioconversion en alcool coniférylique et/ou en acide férulique.

Enfin, l'invention a également pour objet un procédé production d'alcool coniférylique et/ou d'acide férulique naturels dans lequel les étapes b), b1), b2) et b3) sont répétées autant de fois qu'il est souhaité de copies du système d'expression, dans le but d'augmenter le nombre de copies du système d'expression dans le génome de la levure. Selon un mode de réalisation préféré de l'invention, les levures Saccharomyces cerevisiae sont dans un premier temps transformées avec le système d'expression puis sélectionnées comme décrit précédemment. Ces levures sont ensuite transformées avec le vecteur d'excision, de façon à permettre l'élimination du marqueur de sélection comme décrit précédemment. Les levures ainsi obtenues ne possèdent plus de marqueur de sélection dans leur génome et ont intégré au moins une copie du système d'expression.
Dans un second temps, de façon à augmenter le nombre de copies du système d'expression, ces levures sont de nouveau transformées avec le système d'expression. Le même procédé de sélection, de transformation avec le vecteur d'excision et de sélection finale est réalisé comme précédemment. Les levures ainsi obtenues ne possèdent plus de marqueurs de sélection et ont intégré dans leur génome au moins deux copies du système d'expression. Ce procédé peut être renouvelé autant de fois que souhaitées.

La présente invention a pour objet un procédé de production de vanilline comprenant les étapes a), b), c) et d) telles que décrites précédemment et une étape ultérieure de conversion de l'acide férulique et/ou de l'alcool coniférylique, produit à l'étape d), en vanilline.

Selon un mode de réalisation préféré de l'invention, la Saccharomyces cerevisae de part son propre matériel génétique est capable de convertir l'acide férulique produit à l'étape d) en vanilline.

Selon un autre mode de réalisation préféré de l'invention, l'étape de conversion de l'acide férulique et/ou de l'alcool coniférylique en vanilline est réalisée par voie enzymatique ou voie biochimique selon les procédés décrits dans les brevets EP 0 606 441 et EP 0 804 606.

La présente invention sera mieux comprise à l'aide du complément de description qui va suivre, qui se réfère à des exemples d'obtention du système d'expression comprenant la vanillyl alcool oxydase, de vecteurs comprenant ce système d'expression, de vecteurs d'excision, et de leur utilisation pour la production, par bioconversion de l'eugénol, d'alcool coniférylique et d'acide férulique.
Dans les exemples qui suivent, donnés à titre illustratif, il sera fait référence aux figures en annexe, dans lesquelles :
- la figure 1 présente un schéma du système d'expression,
- la figure 2 présente l'expression de la vanillyl alcool oxydase par les levures,
- la figure 3 présente le procédé d'obtention du vecteur d'excision pFL44s-NAT1-CRE.

### Exemple 1 : Expression de la vanillyl alcool oxydase dans la levure par transformation de la levure avec le système d'expression.

### 1/ Synthèse du système d'expression.

Le système d'expression comprend la séquence nucléotidique de 2715 pb décrite dans SEQ ID n°13. Cette séquence nucléotidique a été construite à partir d'une séquence synthétique (SEQ ID n°14) et d'un insert de sélection (SEQ ID n°15).
La séquence synthétique possède une taille de 1225 pb et a été synthétisé par GenScript Corporation. Cette séquence comprend :
- la séquence TY1A
- une séquence LoxP
- la séquence du promoteur du gène TDH3
- une séquence de sites multiples de clonage
- la séquence du terminateur du gène CYC1
- la séquence TY1B
L'insert de sélection a été isolé à partir du vecteur pUG6 cloné dans E. coli (Güldener, U., Heck, S., Fiedler, T., BeinHauer, J., and Hegemann, J.H. 1996. A new efficient gene disruption cassette for repeated use in budding yeast. Nucleic Acids Res. 24:2519-2524). L'insert de sélection comporte 1500 pb et comprend :
- une séquence loxP
- la séquence du promoteur du gène TEF1
- la séquence du gène kan^{r}
- la séquence du terminateur du gène TEF1
Cet insert a été isolé par digestion du vecteur pUG6 avec les enzymes de restriction SalI et SacI.

Le système d'expression comprenant la séquence synthétique a été cloné dans le vecteur pUC57 (GenScript Corporation) grâce aux sites de restriction EcoRI et PstI.

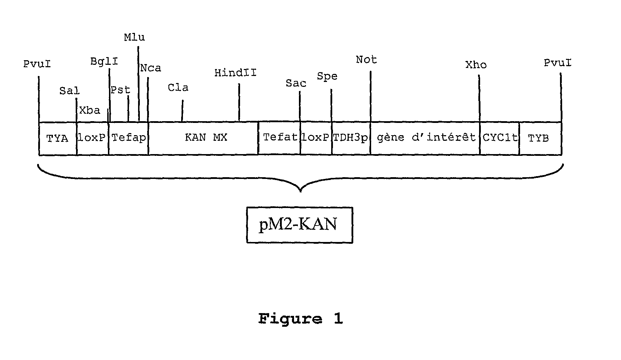

Le vecteur pUG6 et le vecteur pUC57 contenant le système d'expression ont été digérés par les enzymes de restriction SalI et SacI. L'insert de sélection est ensuite inséré par simple ligation dans la séquence synthétique entre TY1A et la séquence LoxP situés de part et d'autre des sites de restriction SalI et SacI.
Le vecteur ainsi obtenu comportant le système d'expression (séquence synthétique + insert de sélection) est appelé pM2-KAN **(****figure 1****)**

Les sites de restriction XbaI et SacI présents dans l'insert de sélection permettent de changer le marqueur de sélection et ses séquences d'expression si l'utilisateur le souhaite.
Les sites de restriction SalI et SpeI permettent d'éliminer ou de remplacer l'ensemble des séquences constitué par le marqueur de sélection et ses séquences d'expression encadrés par les deux séquences LoxP.

### 2/ Insertion du gène codant la vanillyl alcool oxydase dans le système d'expression.

La séquence nucléotidique de la VAO est issue de *Penicillium Simplicissium* (référence Genbank Y15627). La séquence VAO (SEQ ID n°1) utilisée dans cette expérience a été synthétisée avec un site NotI en 5' et XhoI en 3' pour permettre son clonage dans le vecteur pivex, et ceci en phase avec une séquence nucléotidique permettant de produire une protéine avec une étiquette de six histidines en C-terminal.
Le vecteur pivex contenant la séquence VAO telle que décrite ci-dessus a été digéré par NotI et EcoRI defaçon à libérer la séquence VAO-6His. Cette séquence est ensuite clonée par simple ligation dans le vecteur pUC57 contenant le système d'expression digéré préalablement par NotI et MfeI.

### 3/ Transformation des levures avec le système d'expression comprenant le gène codant la vanillyl alcool oxidase.

Le vecteur pUC57 contenant le système d'expression est digéré par l'enzyme de restriction PvuII. Cette enzyme coupe de part et d'autre du système d'expression contenant le gène d'intérêt : la VAO. Le fragment d'ADN issu de cette digestion est purifié et transformé dans les levures selon une méthode de choc thermique en présence de PEG/lithium acétate.
Les levures transformées, grâce à la présence du gène de résistance KANr porté par le système d'expression, sont sélectionnées sur milieu riche YPD (extrait de levure 1%, peptone 1%, glucose 2%) contenant 300 mg/l de Geneticin. Vingt-quatre clones de levures Saccharomyces cerevisiae (souche 92411) ont été obtenus après transformation (clones 92411 KANVAO).

### 3/ Analyse de l'expression de vanillyl alcool oxydase par les levures transformées.

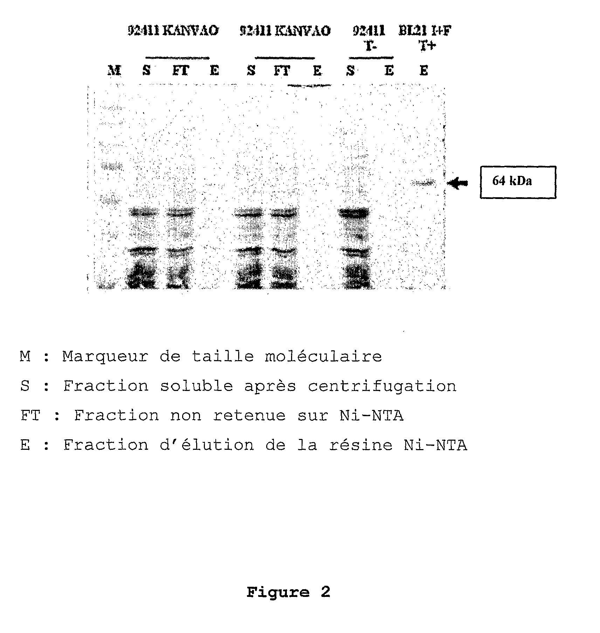

Les levures transformées sont cultivées sur milieu complet Glucosé (YPD). La protéine VAO est ensuite purifiée par affinité sur résine Ni NTA comme dans l'exemple 1. Les protéines retenues sur la résine sont analysées par électrophorèse sur gel de polyacrylamide (gel SDS-PAGE) et détectées au bleu de coomassie. La **figure 2** montre pour deux clones que la protéine VAO est produite (colonnes E 92411 KANVAO). Le clone 92411 T- correspond au témoin négatif, c'est-à-dire à une souche 92411 non transformée, ne produit pas de protéine VAO, tandis que BL21 I+F correspond à un témoin positif résultant du clonage de la vanillyl VAO dans le vecteur pivex et de sa production dans E. Coli.

### Exemple 2 : Production d'alcool coniférylique et d'acide férulique par bioconversion de l'eugénol dans les levures exprimant la vanillyl alcool oxydase.

Les levures transformées avec le système d'expression contenant la vanillyl alcool oxydase, telles que décrites précédemment, ont été sélectionnées tout d'abord pour leur capacité à convertir l'eugénol en alcool coniférylique puis, pour certaines d'entre elles, pour leur capacité à produire de l'acide férulique. Ainsi, le clone 93205, pré-sélectionné pour sa capacité à former de l'alcool coniférylique s'est montré capable par la suite, via ses dérivés, de convertir l'eugénol en acide férulique.
Le clone 93207 et sa descendance, ont été choisis comme exemples de souches capables de convertir l'eugénol en alcool coniférylique en fermenteur.

### 1/ Production d'alcool coniférylique en fermenteur avec la souche 93207 et un de ses dérivés 93334.

Deux spores de la souche 93207 ont été croisées pour donner la souche diploïde 93334, qui comprend ainsi deux copies du système d'expression contenant la vanillyl alcool oxydase.
La souche 93334 est ensuite cultivée dans 100 ml de milieu au malt pendant deux jours pour atteindre une concentration d'environ 3 à 6 x 10⁸ cellules/ml. Les cellules sont ensuite inoculées dans un fermenteur contenant 3 litres de milieu au malt. L'inoculation est effectuée avec la moitié du volume de préculture, à 30°C et sous agitation de 500 rpm. L'aération est portée à 1 litre d'air/minute.
Après 20h de culture, l'eugénol est ajouté en solution dans du glucose 50% (60g d'eugénol + 120 ml de glucose à 50% dans H₂0).
Après 18h de conversion, la fermentation est arrêtée. Un prélèvement est effectué. Il est acidifié avec de l'acide phosphorique, dilué par 2 dans de l'éthanol et centrifugé à 8000 rpm. Le surnageant est analysé en HPLC.

Les résultats montrent qu'en 18 heures l'eugénol est converti en quasi-totalité et que 22g/l d'alcool coniférylique ont été synthétisés (rendement molaire proche de 100 %).

### 2/ Production d'acide férulique en fermenteur avec la souche 93205 et un de ses dérivés 93342.

La souche haploïde 93242, issue de la sporulation de la souche 93205, est cultivée dans 100 ml de milieu au malt pendant deux jours, sous agitation (150 rpm) et à 30°C. Les cellules sont ensuite mises en culture dans un fermenteur contenant 3 litres de milieu au malt. L'inoculation est effectuée avec la moitié du volume de préculture, sous agitation (500 rpm) et à 30°C. L'aération est portée à 0,45 litre d'air/minute pendant 24h.
Après 24h de culture, la solution d'eugénol est ajoutée en continu avec un débit de 0,25 à 0,5 g d'eugénol par heure. Des prélèvements sont effectués régulièrement pour suivre la conversion du substrat en dérivés phénoliques. Chaque prélèvement est acidifié avec de l'acide phosphorique et centrifugé à 8000 rpm. Le surnageant est analysé en HPLC.

**Débit de substrat : 2.5 ml/H Concentrations en gramme par litre**

| temps (heures) | Eugénol distribué | eugénol restant | alcool coniférylique | Acide Férulique |
|---|---|---|---|---|
| 0 | 0 | 0 | 0 | 0 |
| 16 | 1.6 | 0 | 0.7 | 0.75 |
| 40 | 3.8 | 0 | 0 | 3.8 |
| 64 | 7.1 | 0 | 0.2 | 6.7 |
| 112 | 10 | 0 | 0 | 10.11 |

Dans ces conditions, 10 g/l d'acide férulique sont produits à partir de 10 g/l d'eugénol (rendement molaire = 85%).

### Exemple 3 : Excision du marqueur de sélection du génome des levures transformées.

### 1/ Construction du vecteur pFL44s-NAT1-CRE pour l'excision du marqueur de sélection.

Le vecteur d'excision a pour vecteur originel dans cet exemple le vecteur pFL44s (référence Genbank X70266). Ce vecteur d'une taille de 4319 pb possède :
- la séquence du gène URA3
- la séquence du gène de résistance AmpR
- un site multiple de clonage
- la séquence de l'origine de réplication 2 micron.

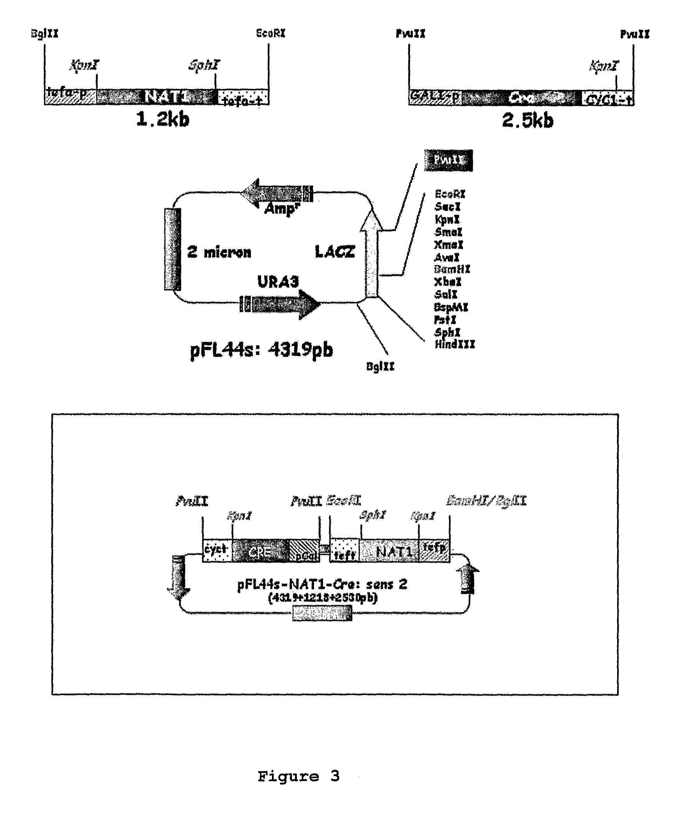

Dans ce vecteur pFL44s sont insérés un insert comprenant un marqueur de sélection et un insert comprenant la séquence de l'enzyme CRE.
L'insert comprenant le marqueur de sélection, ici le gène de résistance à la nourseothricin NAT1, est obtenu à partir du vecteur codant pour la nourseothricin (référence Genbank X73149). Ledit vecteur et le vecteur pFL44s sont digérés par les enzymes de restriction BglII et EcoRI. L'insert NAT1 comprenant le promoteur du gène TEF, la séquence du gène NAT1 et le terminateur du gène TEF, est inséré par simple ligation dans le vecteur pFL44s. Le vecteur ainsi obtenu est le vecteur pFL44s-NAT1.
L'insert comprenant la séquence de l'enzyme CRE est obtenu à partir du vecteur psh47 (référence Genbank AF298782). Le vecteur psh47 et le vecteur pFL44s-NAT1 sont digérés par l'enzyme de restriction PvuII. L'insert CRE comprenant le promoteur du gène GAL1, la séquence de gène CRE et le terminateur du gène CYC1, est ensuite inséré par simple ligation dans le vecteur pFL44s-NAT1. Le vecteur ainsi obtenu est le vecteur pFL44s-NAT1-CRE ayant pour séquence la séquence SEQ ID n°11 (figure 3).

### 2/ Transformation des levures avec le vecteur d'excision pour éliminer le marqueur de sélection présent dans le génome des levures.

Les levures comprenant le système d'expression contenant la vanillyl alcool oxydase, par exemple la souche 93334, sont transformées avec le vecteur d'excision pFL44s-NAT1-CRE. Les levures transformées sont sélectionnées sur milieu riche (YPD) additionné de clonNAT (100 mg/ml).
Les levures sélectionnées positivement sont ensuite cultivées dans un milieu YPGalactose. Le promoteur du gène CRE étant inductible en présence de Galactose, cette condition de culture permet l'induction de l'expression de l'enzyme CRE dans les levures.
Les résultats de cette expérience montrent que 80% des clones ont perdu le marqueur KanR.
Le procédé de conversion d'eugénol en alcool coniférylique tel que décrit en 1/ est réalisé avec une souche 93334 ayant perdu le marqueur de sélection. Après 18h de conversion, les mêmes résultats sont obtenus, suggérant que la perte du marqueur de sélection n'affecte pas la capacité de bioconversion de la souche.

## Revendications

1. Levure appartenant à l'espèce *Saccharomyces cerevisiae* transformée par le gène codant la vanillyl alcool oxydase ayant pour séquence la séquence SEQ ID n°1 ou toute séquence identique à au moins 70%, de préférence 80%, très préférentiellement à 90% à la séquence SEQ ID n°1.

2. Levure selon la revendication **1, caractérisée en ce que** le gène codant la vanillyl alcool oxydase est contenu dans un système d'expression, ledit système comprenant :
(1) des moyens visant à l'intégration dudit système dans le génome de ladite levure, comprenant deux séquences nucléotidiques,
(2) des moyens visant à la sélection de ladite levure ayant intégré ledit système, comprenant un insert de sélection comprenant deux séquences LoxP correspondant à la séquence SEQ ID n°3 encadrant un promoteur, un marqueur de sélection du type gène de résistance aux antibiotiques et un terminateur,
(3) une cassette d'expression du gène codant la vanillyl alcool oxydase, comprenant un promoteur permettant l'expression dudit gène, au moins un site de clonage permettant l'intégration du gène, et un terminateur.

3. Levure selon la revendication **2, caractérisée en ce que** les séquences nucléotidiques d'intégration dudit système d'expression dans le génome de la levure sont les séquences TY1A correspondant à la séquence SEQ ID n°4 et TY1B correspondant à la séquence SEQ ID n°5 de *Saccharomyces cerevisiae* ou toutes séquences qui leur sont identiques à au moins 70%, de préférence 80%, très préférentiellement 90%.

4. Levure selon l'une des revendications **2** à **3, caractérisée en ce que** le promoteur permettant l'expression de la vanillyl alcool oxydase est le promoteur du gène TDH3 (SEQ ID n°6) de *Saccharomyces cerevisiae,* et le terminateur associé est le terminateur du gène CYC1 correspondant à la séquence SEQ ID n°7 de *Saccharomyces cerevisiae* ou toutes séquences qui leur sont identiques à au moins 70%, de préférence 80%, très préférentiellement 90%.

5. Levure selon l'une des revendications **2** à **4, caractérisée en ce que** le promoteur et le terminateur permettant l'expression du marqueur de sélection sont le promoteur correspondant à la séquence SEQ ID n°8 et le terminateur correspondant à la séquence SEQ ID n°9 du gène TEF1 de Ashbya gossypii ou toutes séquences qui leur sont identiques à au moins 70%, de préférence 80%, très préférentiellement 90%.

6. Levure selon l'une des revendications **2 à 5, caractérisée en ce que** le marqueur de sélection est un gène de résistance aux antibiotiques choisi dans le groupe comprenant les gènes de résistance à la généticine, nourseothricine, phléomycine, zéocine ou tout autre gène de résistance à un antibiotique dominant pour lequel la levure sauvage est sensible et est de préférence le gène de résistance à la généticine correspondant à la séquence SEQ ID n°10 ou toute séquence qui lui est identique à au moins 70%, de préférence 80%, très préférentiellement 90%.

7. Levure selon l'une des revendications **2** à **6,** dans laquelle le système d'expression contenant la vanillyl alcool oxydase a pour séquence la séquence nucléotidique SEQ ID n°11 ou toute séquence qui lui est identique à au moins 70%, de préférence 80%, très préférentiellement 90%.

8. Procédé de production d'alcool coniférylique et/ou d'acide férulique naturels, comprenant les étapes suivantes :
a) clonage de la séquence nucléotidique du gène codant la vanillyl alcool oxydase tel que défini dans la revendication 1, dans le système d'expression tel que défini dans l'une des revendications **2** à **7,**
b) transformation d'une levure appartenant à l'espèce *Saccharomyces cerevisiae* avec le système d'expression ainsi obtenu,
c) culture de la levure dans les conditions permettant l'expression de la vanillyl alcool oxydase,
d) mise en contact de la levure avec de l'eugénol.

9. Procédé selon la revendication **8, caractérisé en ce qu'**il permet la production d'acide férulique.

10. Procédé selon l'une quelconque des revendications **8** à **9,** comprenant après l'étape b) et avant les étapes c) et d), les étapes b1), b2) et b3) suivantes :
b1) transformation de la levure avec un vecteur d'excision choisi parmi les vecteurs multicopies, de préférence pFL44s, comprenant la séquence SEQ ID n°12 ou toute séquence identique à au moins 70%, de préférence 80%, très préférentiellement 90%, à la séquence SEQ ID n°12,
b2) culture de la levure dans les conditions permettant l'expression de l'enzyme CRE,
b3) isolement des levures ayant perdu le marqueur de sélection.

11. Procédé selon la revendication **10,** dans lequel les étapes b), b1), b2) et b3) sont répétées autant de fois qu'il est souhaité de copies du système d'expression.

12. Procédé de production de vanilline comprenant les étapes a), b), c) et d) telles que décrites dans l'une quelconque des revendications **8** à **11** et une étape ultérieure de conversion de l'acide férulique et/ou de l'alcool coniférylique produits à l'étape d) en vanilline.

13. Procédé de production de vanilline selon la revendication **12** dans lequel l'étape de conversion de l'acide férulique et/ou de l'alcool coniférylique produits à l'étape d) en vanilline s'effectue dans une levure ou par voie biochimique ou voie enzymatique.

## Patentansprüche

1. Zur Art *Saccharomyces cerevisiae* gehörende Hefe, die mit dem Gen, das für die Vanillylalkoholoxidase codiert und als Sequenz die Sequenz SEQ ID NO. 1 oder jegliche Sequenz mit mindestens 70%, vorzugsweise 80%, stark bevorzugt 90% Identität zu der Sequenz von SEQ ID NO. 1 aufweist, transformiert ist.

2. Hefe nach Anspruch 1, **dadurch gekennzeichnet, dass** das Gen, das für die Vanillylalkoholoxidase codiert, in einem Expressionssystem enthalten ist, wobei das System Folgendes umfasst:
(1) Mittel zum Integrieren des Systems in das Genom der Hefe, die zwei Nukleotidsequenzen umfassen,
(2) Mittel zur Selektion der Hefe, die in das System integriert hat, die ein Selektionsinsert umfassen, das zwei LoxP-Sequenzen entsprechend der Sequenz SEQ ID NO. 3 umfasst, die einen Promoter, einen Selektionsmarker des Antibiotikaresistenzgen-Typs und einen Terminator umrahmen,
(3) eine Expressionskassette des Gens, das für die Vanillylalkoholoxidase codiert, die einen Promoter, der die Expression des Gens gestattet, mindestens eine Klonierungsstelle, die die Integration des Gens gestattet, und einen Terminator umfasst.

3. Hefe nach Anspruch 2, **dadurch gekennzeichnet, dass** es sich bei den Integrationsnukleotidsequenzen des Expressionssystems in dem Hefegenom um die Sequenzen TY1A entsprechend der Sequenz SEQ ID NO. 4 und TY1 B entsprechend der Sequenz SEQ ID NO. 5 von *Saccharomyces cerevisiae* oder allen Sequenzen, die mit ihnen zu mindestens 70%, vorzugsweise 80%, stark bevorzugt 90%, identisch sind, handelt.

4. Hefe nach einem der Ansprüche 2 bis 3, **dadurch gekennzeichnet, dass** es sich bei dem Promoter, der die Expression der Vanillylalkoholoxidase gestattet, um den Promoter des TDH3-Gens (SEQ ID NO. 6) von *Saccharomyces cerevisiae* handelt und es sich bei dem damit assoziierten Terminator um den Terminator des CYC1-Gens entsprechend der Sequenz SEQ ID NO. 7 von *Saccharomyces cerevisiae* oder allen Sequenzen, die mit ihnen zu mindestens 70%, vorzugsweise 80%, stark bevorzugt 90%, identisch sind, handelt.

5. Hefe nach einem der Ansprüche 2 bis 4, **dadurch gekennzeichnet, dass** es sich bei dem Promoter und dem Terminator, die die Expression des Selektionsmarkers gestatten, um den Promoter entsprechend der Sequenz SEQ ID NO. 8 und den Terminator entsprechend der Sequenz SEQ ID NO. 9 des TEF1-Gens von Ashbya gossypii oder allen Sequenzen, die mit ihnen zu mindestens 70%, vorzugsweise 80%, stark bevorzugt 90%, identisch sind, handelt.

6. Hefe nach einem der Ansprüche 2 bis 5, **dadurch gekennzeichnet, dass** es sich bei dem Selektionsmarker um ein Antibiotikaresistenzgen, ausgewählt aus der Gruppe umfassend die Gene für Resistenz gegen Geneticin, Nourseothricin, Phleomycin, Zeocin oder jegliches andere Gen für Resistenz gegen ein dominantes Antibiotikum, gegenüber dem die Wildhefe anfällig ist, und vorzugsweise um das Geneticinresistenzgen entsprechend der Sequenz SEQ ID NO. 10 oder jeglicher Sequenz, die zu ihr zu mindestens 70%, vorzugsweise 80%, stark bevorzugt 90% identisch ist, handelt.

7. Hefe nach einem der Ansprüche 2 bis 6, wobei das Expressionssystem, das die Vanillylalkoholoxidase enthält, als Sequenz die Nukleotidsequenz SEQ ID NO. 11 oder jegliche Sequenz, die zu ihr zu mindestens 70%, vorzugsweise 80%, stark bevorzugt 90% identisch ist, aufweist.

8. Verfahren zur Produktion von natürlichem Coniferylalkohol und/oder natürlicher Ferulasäure, umfassend die folgenden Schritte:
a) Klonieren der Nukleotidsequenz des wie in Anspruch **1** definierten Gens, das für die Vanillylalkoholoxidase codiert, in das wie in einem der Ansprüche 2 bis 7 definierte Expressionssystem,
b) Transformieren einer zur Art *Saccharomyces cerevisiae* gehörenden Hefe mit dem so erhaltenen Expressionssystem,
c) Kultivieren der Hefe unter Bedingungen, die die Expression der Vanillylalkoholoxidase gestatten,
d) Inkontaktbringen der Hefe mit Eugenol.

9. Verfahren nach Anspruch 8, **dadurch gekennzeichnet, dass** es die Produktion von Ferulasäure gestattet.

10. Verfahren nach einem der Ansprüche 8 bis 9, das nach Schritt b) und vor den Schritten c) und d) die folgenden Schritte b1), b2) und b3) umfasst:
b1) Transformieren der Hefe mit einem Exzisionsvektor, der aus Multicopy-Vektoren, vorzugsweise pFL44s-Vektoren, ausgewählt ist und der die Sequenz SEQ ID NO. 12 oder jegliche Sequenz, die zu der Sequenz SEQ ID NO. 12 zu mindestens 70%, vorzugsweise 80%, stark bevorzugt 90% identisch ist, umfasst,
b2) Kultivieren der Hefe unter Bedingungen, die die Expression des CRE-Enzyms gestatten,
b3) Isolieren der Hefen, die den Selektionsmarker verloren haben.

11. Verfahren nach Anspruch 10, wobei die Schritte b), b1), b2) und b3) so oft wiederholt werden, wie Kopien des Expressionssystems gewünscht werden.

12. Verfahren zur Produktion von Vanillin, umfassend die Schritte a), b), c) und d) wie in einem der Ansprüche 8 bis 11 beschrieben sowie einen weiteren Schritt der Umwandlung der in Schritt d) produzierten Ferulasäure bzw. des in Schritt d) produzierten Coniferylalkohols in Vanillin.

13. Verfahren zur Produktion von Vanillin nach Anspruch 12, wobei der Schritt der Umwandlung der in Schritt d) produzierten Ferulasäure bzw. des in Schritt d) produzierten Coniferylalkohols in Vanillin in einer Hefe oder auf biochemischem oder enzymatischem Weg erfolgt.

## Claims

1. A yeast belonging to *Saccharomyces cerevisiae* transformed by the gene encoding vanillyl alcohol oxidase, the sequence of which is the sequence SEQ ID No. 1 or any sequence having at least 70%, preferably 80%, very preferably 90%, identity with the sequence SEQ ID No. 1.

2. The yeast as claimed in claim **1, characterized in that** the gene encoding vanillyl alcohol oxidase is contained in an expression system, said system comprising:
(1) means for integrating said system into the genome of said yeast, comprising two nucleotide sequences,
(2) means for selecting said yeast having integrated said system, comprising a selection insert comprising two LoxP sequences corresponding to the sequence SEQ ID No. 3, bordering a promoter, a selectable marker of the gene for resistance to antibiotics type, and a terminator,
(3) an expression cassette for the gene encoding vanillyl alcohol oxidase, comprising a promoter which allows the expression of said gene, at least one cloning site which allows the integration of the gene, and a terminator.

3. The yeast as claimed in claim **2, characterized in that** the nucleotide sequences for integration of said expression system into the genome of the yeast are the TY1A sequences corresponding to the sequence SEQ ID No. 4 and the TY1B sequence corresponding to the sequence SEQ ID No. 5, of *Saccharomyces cerevisiae,* or any sequences which are at least 70%, preferably 80%, very preferably 90%, identical thereto.

4. The yeast as claimed in either of claims **2** and **3, characterized in that** the promoter which allows the expression of the vanillyl alcohol oxidase is the promoter of the *Saccharomyces cerevisiae* TDH3 gene (SEQ ID No. 6), and the associated terminator is the terminator of the *Saccharomyces cerevisiae* CYC1 gene, corresponding to the sequence SEQ ID No. 7 or any sequences which are at least 70%, preferably 80%, very preferably 90%, identical thereto.

5. The yeast as claimed in one of claims **2** to **4, characterized in that** the promoter and the terminator which allow the expression of the selectable marker are the promoter corresponding to the sequence SEQ ID No. 8 and the terminator corresponding to the sequence SEQ ID No. 9 of the *Ashbya gossypii* TEF1 gene or any sequences which are at least 70%, preferably 80%, very preferably 90%, identical thereto.

6. The yeast as claimed in one of claims **2** to **5, characterized in that** the selectable marker is a gene for resistance to antibiotics, chosen from the group comprising the genes for resistance to geneticin, nourseothricin, phleomycin or zeocin or any other gene for resistance to a dominant antibiotic to which the wild-type yeast is sensitive, and is preferably the geneticin-resistance gene corresponding to the sequence SEQ ID No. 10 or any sequence which is at least 70%, preferably 80%, very preferably 90%, identical thereto.

7. The yeast as claimed in one of claims **2** to **6,** in which the sequence of the expression system containing vanillyl alcohol oxidase is the nucleotide sequence SEQ ID No. 11 or any sequence which is at least 70%, preferably 80%, very preferably 90%, identical thereto.

8. A method for producing natural coniferyl alcohol and/or natural ferulic acid, comprising the following steps:
a) cloning the nucleotide sequence of the gene encoding vanillyl alcohol oxidase as defined in claim 1, into the expression system as defined in one of claims **2** to **7,**
b) transforming a yeast belonging to *Saccharomyces cerevisiae* with the expression system thus obtained,
c) culturing the yeast under the conditions which allow the expression of the vanillyl alcohol oxidase,
d) bringing the yeast into contact with eugenol.

9. The method as claimed in claim 8, **characterized in that** it allows the production of ferulic acid.

10. The method as claimed in any one of claims **8** to **9,** comprising, after step b) and before steps c) and d), the following steps b1), b2) and b3):
b1) transforming the yeast with the excision vector chosen from multicopy vectors, preferably pFL44s, comprising the sequence SEQ ID No. 12 or any sequence at least 70%, preferably 80%, very preferably 90%, identical with the sequence SEQ ID No. 12,
b2) culturing the yeast under the conditions which allow the expression of the CRE enzyme,
b3) isolating the yeasts having lost the selectable marker.

11. The method as claimed in claim **10,** in which steps b), b1), b2) and b3) are repeated as many times as the desired number of copies of the expression system.

12. A method for producing vanillin, comprising steps a), b), c) and d) as described in any one of claims **8** to **11** and a subsequent step of converting the ferulic acid and/or the coniferyl alcohol, produced in step d), to vanillin.

13. The method for producing vanillin as claimed in claim **12,** in which the step of converting the ferulic acid and/or the coniferyl alcohol, produced in step d), to vanillin is carried out in a yeast or by biochemical process or enzymatic process.


### SEQUENCE LISTING

<110> MANE, Jean
<120> système de production de molécules aromatiques par bioconversion
<130> BFF060094
<160> 15
<170> PatentIn version 3.3
<210> 1
   <211> 1734
   <212> DNA
   <213> P. simplicissimum
<400> 1
   atgagcggcc gctcaaagac tcaagagttc agaccattga ctttgccacc aaagttgtct      60
   ttgtcagact tcaacgagtt cattcaagac attattagaa ttgttggttc agagaacgtt     120
   gaggttattt catctaagga ccaaattgtt gacggttctt atatgaagcc aactcatact     180
   catgacccac atcatgttat ggaccaagac tatttcttgg cttctgctat tgttgctcca     240
   agaaacgttg ctgacgttca atctattgtt ggtttggcta acaagttctc tttcccattg     300
   tggccaattt caattggtag aaactctggt tatggtggtg ctgctccccg ggtttcaggt     360
   tctgttgttt tggacatggg taagaacatg aacagagttt tggaggttaa cgttgaaggt     420
   gcttattgtg ttgttgaacc aggtgttact tatcatgact tgcataacta tttggaggct     480
   aacaacttga gagacaaatt gtggttagac gttccagatc taggtggtgg ttctgttttg     540
   ggtaacgctg ttgaaagagg tgttggttat actccatacg gtgatcattg gatgatgcat     600
   tctggtatgg aggttgtttt ggctaacggt gagttgttaa gaactggtat gggtgcttta     660
   ccagacccaa aaagaccaga gactatgggt ttgaagccag aagatcaacc atggtctaag     720
   attgctcatt tgttcccata cggtttcggt ccatatattg atggtttgtt ctctcaatct     780
   aacatgggta ttgttactaa gattggtatt tggttaatgc caaacccagg tggttatcaa     840
   tcttatttga ttactttacc aaaggatggt gacttgaagc aagctgttga cattattaga     900
   ccattgagat taggtatggc tttgcaaaac gttccaacta ttagacatat tttattggat     960
   gctgctgttt tgggtgacaa gagatcatat tcttcaaaaa ctgagccatt gtctgacgag    1020
   gagttggaca agattgctaa gcaattgaac ttgggtagat ggaactttta tggtgctttg    1080
   tatggtccag aaccaattag aagagttttg tgggagacta ttaaagatgc tttctctgct    1140
   attccaggtg ttaagttcta ttttccagaa gatactccag agaactctgt tttgagagtt    1200
   agagacaaga ctatgcaagg tattccaact tatgacgagt taaaatggat tgactggttg    1260
   ccaaacggtg ctcatttgtt cttctctcca attgctaagg tttctggtga agacgctatg    1320
   atgcaatatg ctgttactaa aaagagatgt caagaggctg gtttggactt cattggtact    1380
   tttactgttg gcatgcgtga gatgcatcat attgtttgta ttgttttcaa caagaaggac    1440
   ttgattcaaa agagaaaggt tcaatggttg atgagaactt tgattgatga ctgtgctgct    1500
   aatggttggg gtgagtatag aactcatttg gctttcatgg accaaattat ggagacttat    1560
   aactggaaca actcttcatt cttgagattc aacgaggttt tgaagaacgc tgttgatcca    1620
   aacggtatta ttgctccagg taagtcaggt gtttggccat ctcaatattc tcatgttact    1680
   tggaagctcg agcgagctcc cggggggggt tctcatcatc atcatcatca ttaa          1734
<210> 2
   <211> 29
   <212> DNA
   <213> artificial
<220>
   <223> multiple cloning site
<400> 2
   gcggccgcgg atcccaattg cgactcgag 29
<210> 3
   <211> 34
   <212> DNA
   <213> bacteriophage P1
<400> 3
   ataacttcgt ataatgtatg ctatacgaag ttat 34
<210> 4
   <211> 149
   <212> DNA
   <213> S. cerevisiae
<400> 4
   ctgtgcttcg gttacttcta aggaagtcca cacaaatcaa gatccgttag acgtttcagc 60
   ttccaaaaca gaagaatgtg agaaggcttc cactaaggct aactctcaac agacaacaac 120
   acctgcttca tctgctgttc cagagaacc 149
<210> 5
   <211> 153
   <212> DNA
   <213> S. cerevisiae
<400> 5
   acctgataca agaacttaac aagaaaccaa ttattaaagg cttacttact gatagtagat 60
   caacgatcag tataattaag tctacaaatg aagagaaatt tagaaacaga ttttttggca 120
   caaaggcaat gagacttaga gatgaagtat cag 153
<210> 6
   <211> 596
   <212> DNA
   <213> S. cerevisiae
<400> 6
   tcaaaaaact agtcttttaa ttctgctgta acccgtacat gcccaaaata gggggcgggt 60
   tacacagaat atataacatc gtaggtgtct gggtgaacag tttattcctg gcatccacta 120
   aatataatgg agcccgcttt ttaagctggc atccagaaaa aaaaagaatc ccagcaccaa 180
   aatattgttt tcttcaccaa ccatcagttc ataggtccat tctcttagcg caactacaga 240
   gaacaggggc acaaacaggc aaaaaacggg cacaacctca atggagtgat gcaacctgcc 300
   tggagtaaat gatgacacaa ggcaattcac ccacgcatgt atctatctca ttttcttaca 360
   ccttctatta ccttctgctc tctctgattt ggaaaaagct gaaaaaaaag gttgaaacca 420
   gttccctgaa attattcccc tacttgacta ataagtatat aaagacggta ggtattgatt 480
   gtaattctgt aaatctattt cttaaacttc ttaaattcta cttttatagt tagtcttttt 540
   tttagtttta aaacaccaag aacttagttt cgaataaaca cacataaaca aacaaa 596
<210> 7
   <211> 204
   <212> DNA
   <213> S. cerevisiae
<400> 7
   acaggcccct tttcctttgt cgatctcatg taattagtta tgtcacgctt acattcacgc 60
   cctcctccca catccgctct aaccgaaaag gaaggagtta gacaacctga agtctaggtc 120
   cctatttatt ttttttaata gttatgttag tattaagaac gttatttata tttcaaattt 180
   ttcttttttt tctgtacaaa cgcg 204
<210> 8
   <211> 383
   <212> DNA
   <213> A. gossypii
<400> 8
   gtttagcttg cctcgtcccc gccgggtcac ccggccagcg acatggaggc ccagaatacc 60
   ctccttgaca gtcttgacgt gcgcagctca ggggcatgat gtgactgtcg cccgtacatt 120
   tagcccatac atccccatgt ataatcattt gcatccatac attttgatgg ccgcacggcg 180
   cgaagcaaaa attacggctc ctcgctgcag acctgcgagc agggaaacgc tcccctcaca 240
   gacgcgttga attgtcccca cgccgcgccc ctgtagagaa atataaaagg ttaggatttg 300
   ccactgaggt tcttctttca tatacttcct tttaaaatct tgctaggata cagttctcac 360
   atcacatccg aacataaaca acc 383
<210> 9
   <211> 244
   <212> DNA
   <213> A. gossypii
<400> 9
   tcagtactga caataaaaag attcttgttt tcaagaactt gtcatttgta tagttttttt 60
   atattgtagt tgttctattt taatcaaatg ttagcgtgat ttatattttt tttcgcctcg 120
   acatcatctg cccagatgcg aagttaagtg cgcagaaagt aatatcatgc gtcaatcgta 180
   tgtgaatgct ggtcgctata ctgctgtcga ttcgatacta acgccgccat ccagtgtcga 240
   aaac 244
<210> 10
   <211> 810
   <212> DNA
   <213> E. coli
<400> 10
   atgggtaagg aaaagactca cgtttcgagg ccgcgattaa attccaacat ggatgctgat 60
   ttatatgggt ataaatgggc tcgcgataat gtcgggcaat caggtgcgac aatctatcga 120
   ttgtatggga agcccgatgc gccagagttg tttctgaaac atggcaaagg tagcgttgcc 180
   aatgatgtta cagatgagat ggtcagacta aactggctga cggaatttat gcctcttccg 240
   accatcaagc attttatccg tactcctgat gatgcatggt tactcaccac tgcgatcccc 300
   ggcaaaacag cattccaggt attagaagaa tatcctgatt caggtgaaaa tattgttgat 360
   gcgctggcag tgttcctgcg ccggttgcat tcgattcctg tttgtaattg tccttttaac 420
   agcgatcgcg tatttcgtct cgctcaggcg caatcacgaa tgaataacgg tttggttgat 480
   gcgagtgatt ttgatgacga gcgtaatggc tggcctgttg aacaagtctg gaaagaaatg 540
   cataagcttt tgccattctc accggattca gtcgtcactc atggtgattt ctcacttgat 600
   aaccttattt ttgacgaggg gaaattaata ggttgtattg atgttggacg agtcggaatc 660
   gcagaccgat accaggatct tgccatccta tggaactgcc tcggtgagtt ttctccttca 720
   ttacagaaac ggctttttca aaaatatggt attgataatc ctgatatgaa taaattgcag 780
   tttcatttga tgctcgatga gtttttctaa 810
<210> 11
   <211> 3745
   <212> DNA
   <213> artificial
<220>
   <223> expression system comprising VAO gene
<400> 11
   gaattccagc tgtgcttcgg ttacttctaa ggaagtccac acaaatcaag atccgttaga    60
   cgtttcagct tccaaaacag aagaatgtga gaaggcttcc actaaggcta actctcaaca   120
   gacaacaaca cctgcttcat ctgctgttcc agagaaccga taacttcgta taatgtatgc   180
   tatacgaagt tatatgggta aggaaaagac tcacgtttcg aggccgcgat taaattccaa   240
   catggatgct gatttatatg ggtataaatg ggctcgcgat aatgtcgggc aatcaggtgc   300
   gacaatctat cgattgtatg ggaagcccga tgcgccagag ttgtttctga aacatggcaa   360
   aggtagcgtt gccaatgatg ttacagatga gatggtcaga ctaaactggc tgacggaatt   420
   tatgcctctt ccgaccatca agcattttat ccgtactcct gatgatgcat ggttactcac   480
   cactgcgatc cccggcaaaa cagcattcca ggtattagaa gaatatcctg attcaggtga   540
   aaatattgtt gatgcgctgg cagtgttcct gcgccggttg cattcgattc ctgtttgtaa   600
   ttgtcctttt aacagcgatc gcgtatttcg tctcgctcag gcgcaatcac gaatgaataa   660
   cggtttggtt gatgcgagtg attttgatga cgagcgtaat ggctggcctg ttgaacaagt   720
   ctggaaagaa atgcataagc ttttgccatt ctcaccggat tcagtcgtca ctcatggtga 780
   tttctcactt gataacctta tttttgacga ggggaaatta ataggttgta ttgatgttgg 840
   acgagtcgga atcgcagacc gataccagga tcttgccatc ctatggaact gcctcggtga 900
   gttttctcct tcattacaga aacggctttt tcaaaaatat ggtattgata atcctgatat 960
   gaataaattg cagtttcatt tgatgctcga tgagtttttc taacaaccct taatataact 1020
   tcgtataatg tatgctatac gaagttatta ggtcaaaaaa ctagtctttt aattctgctg 1080
   taacccgtac atgcccaaaa tagggggcgg gttacacaga atatataaca tcgtaggtgt 1140
   ctgggtgaac agtttattcc tggcatccac taaatataat ggagcccgct ttttaagctg 1200
   gcatccagaa aaaaaaagaa tcccagcacc aaaatattgt tttcttcacc aaccatcagt 1260
   tcataggtcc attctcttag cgcaactaca gagaacaggg gcacaaacag gcaaaaaacg 1320
   ggcacaacct caatggagtg atgcaacctg cctggagtaa atgatgacac aaggcaattc 1380
   acccacgcat gtatctatct cattttctta caccttctat taccttctgc tctctctgat 1440
   ttggaaaaag ctgaaaaaaa aggttgaaac cagttccctg aaattattcc cctacttgac 1500
   taataagtat ataaagacgg taggtattga ttgtaattct gtaaatctat ttcttaaact 1560
   tcttaaattc tacttttata gttagtcttt tttttagttt taaaacacca agaacttagt 1620
   ttcgaataaa cacacataaa caaacaaaat gagcggccgc tcaaagactc aagagttcag 1680
   accattgact ttgccaccaa agttgtcttt gtcagacttc aacgagttca ttcaagacat 1740
   tattagaatt gttggttcag agaacgttga ggttatttca tctaaggacc aaattgttga 1800
   cggttcttat atgaagccaa ctcatactca tgacccacat catgttatgg accaagacta 1860
   tttcttggct tctgctattg ttgctccaag aaacgttgct gacgttcaat ctattgttgg 1920
   tttggctaac aagttctctt tcccattgtg gccaatttca attggtagaa actctggtta 1980
   tggtggtgct gctccccggg tttcaggttc tgttgttttg gacatgggta agaacatgaa 2040
   cagagttttg gaggttaacg ttgaaggtgc ttattgtgtt gttgaaccag gtgttactta 2100
   tcatgacttg cataactatt tggaggctaa caacttgaga gacaaattgt ggttagacgt 2160
   tccagatcta ggtggtggtt ctgttttggg taacgctgtt gaaagaggtg ttggttatac 2220
   tccatacggt gatcattgga tgatgcattc tggtatggag gttgttttgg ctaacggtga 2280
   gttgttaaga actggtatgg gtgctttacc agacccaaaa agaccagaga ctatgggttt 2340
   gaagccagaa gatcaaccat ggtctaagat tgctcatttg ttcccatacg gtttcggtcc 2400
   atatattgat ggtttgttct ctcaatctaa catgggtatt gttactaaga ttggtatttg 2460
   gttaatgcca aacccaggtg gttatcaatc ttatttgatt actttaccaa aggatggtga 2520
   cttgaagcaa gctgttgaca ttattagacc attgagatta ggtatggctt tgcaaaacgt 2580
   tccaactatt agacatattt tattggatgc tgctgttttg ggtgacaaga gatcatattc 2640
   ttcaaaaact gagccattgt ctgacgagga gttggacaag attgctaagc aattgaactt 2700
   gggtagatgg aacttttatg gtgctttgta tggtccagaa ccaattagaa gagttttgtg 2760
   ggagactatt aaagatgctt tctctgctat tccaggtgtt aagttctatt ttccagaaga 2820
   tactccagag aactctgttt tgagagttag agacaagact atgcaaggta ttccaactta 2880
   tgacgagtta aaatggattg actggttgcc aaacggtgct catttgttct tctctccaat 2940
   tgctaaggtt tctggtgaag acgctatgat gcaatatgct gttactaaaa agagatgtca 3000
   agaggctggt ttggacttca ttggtacttt tactgttggc atgcgtgaga tgcatcatat 3060
   tgtttgtatt gttttcaaca agaaggactt gattcaaaag agaaaggttc aatggttgat 3120
   gagaactttg attgatgact gtgctgctaa tggttggggt gagtatagaa ctcatttggc 3180
   tttcatggac caaattatgg agacttataa ctggaacaac tcttcattct tgagattcaa 3240
   cgaggttttg aagaacgctg ttgatccaaa cggtattatt gctccaggta agtcaggtgt 3300
   ttggccatct caatattctc atgttacttg gaagctcgag catcatcatc atcatcatga 3360
   attgcgactc gagtaataaa caggcccctt ttcctttgtc gatctcatgt aattagttat 3420
   gtcacgctta cattcacgcc ctcctcccac atccgctcta accgaaaagg aaggagttag 3480
   acaacctgaa gtctaggtcc ctatttattt tttttaatag ttatgttagt attaagaacg 3540
   ttatttatat ttcaaatttt tctttttttt ctgtacaaac gcgacctgat acaagaactt 3600
   aacaagaaac caattattaa aggcttactt actgatagta gatcaacgat cagtataatt 3660
   aagtctacaa atgaagagaa atttagaaac agattttttg gcacaaaggc aatgagactt 3720
   agagatgaag tatcagctgc tgcag 3745
<210> 12
   <211> 3833
   <212> DNA
   <213> artificial
<220>
   <223> NAT1-CRE sequence
<400> 12
   ggatctgttt agcttgcctc gtccccgccg ggtcacccgg ccagcgacat ggaggcccag     60
   aataccctcc ttgacagtct tgacgtgcgc agctcagggg catgatgtga ctgtcgcccg    120
   tacatttagc ccatacatcc ccatgtataa tcatttgcat ccatacattt tgatggccgc    180
   acggcgcgaa gcaaaaatta cggctcctcg ctgcagacct gcgagcaggg aaacgctccc    240
   ctcacagacg cgttgaattg tccccacgcc gcgcccctgt agagaaatat aaaaggttag    300
   gatttgccac tgaggttctt ctttcatata cttcctttta aaatcttgct aggatacagt    360
   tctcacatca catccgaaca taaacaacca tgaccactct tgacgacacg gcttaccggt    420
   accgcaccag tgtcccgggg gacgccgagg ccatcgaggc actggatggg tccttcacca    480
   ccgacaccgt cttccgcgtc accgccaccg gggacggctt caccctgcgg gaggtgccgg    540
   tggacccgcc cctgaccaag gtgttccccg acgacgaatc ggacgacgaa tcggacgccg    600
   gggaggacgg cgacccggac tcccggacgt tcgtcgcgta cggggacgac ggcgacctgg    660
   cgggcttcgt ggtcgtctcg tactccggct ggaaccgccg gctgaccgtc gaggacatcg    720
   aggtcgcccc ggagcaccgg gggcacgggg tcgggcgcgc gttgatgggg ctcgcgacgg 780
   agttcgcccg cgagcggggc gccgggcacc tctggctgga ggtcaccaac gtcaacgcac 840
   cggcgatcca cgcgtaccgg cggatggggt tcaccctctg cggcctggac accgccctgt 900
   acgacggcac cgcctcggac ggcgagcagg cgctctacat gagcatgccc tgcccctaat 960
   cagtactgac aataaaaaga ttcttgtttt caagaacttg tcatttgtat agttttttta 1020
   tattgtagtt gttctatttt aatcaaatgt tagcgtgatt tatatttttt ttcgcctcga 1080
   catcatctgc ccagatgcga agttaagtgc gcagaaagta atatcatgcg tcaatcgtat 1140
   gtgaatgctg gtcgctatac tgctgtcgat tcgatactaa cgccgccatc cagtgtcgaa 1200
   aacgagctcg aattcactgg ccgtcgtttt acaacgtcgt gactgggaaa accctggcgt 1260
   tacccaactt aatcgccttg cagcacatcc ccctttcgcc agctggcacg acaggtttcc 1320
   cgactggaaa gcgggcagtg agcgcaacgc aattaatgtg agttacctca ctcattaggc 1380
   accccaggct ttacacttta tgcttccggc tcctatgttg tgtggaattg tgagcggata 1440
   acaatttcac acaggaaaca gctatgacca tgattacgcc aagcgcgcaa ttaaccctca 1500
   ctaaagggaa caaaagctgg agctctagta cggattagaa gccgccgagc gggtgacagc 1560
   cctccgaagg aagactctcc tccgtgcgtc ctcgtcttca ccggtcgcgt tcctgaaacg 1620
   cagatgtgcc tcgcgccgca ctgctccgaa caataaagat tctacaatac tagcttttat 1680
   ggttatgaag aggaaaaatt ggcagtaacc tggccccaca aaccttcaaa tgaacgaatc 1740
   aaattaacaa ccataggatg ataatgcgat tagtttttta gccttatttc tggggtaatt 1800
   aatcagcgaa gcgatgattt ttgatctatt aacagatata taaatgcaaa aactgcataa 1860
   ccactttaac taatactttc aacattttcg gtttgtatta cttcttattc aaatgtaata 1920
   aaagtatcaa caaaaaattg ttaatatacc tctatacttt aacgtcaagg agaaaaaacc 1980
   ccggattcta gaactagtgg atcccccggg ctgcaggaat tcgatatcaa gcttatcgat 2040
   accgtcgagg ggcagagccg atcctgtaca ctttacttaa aaccattatc tgagtgttaa 2100
   atgtccaatt tactgaccgt acaccaaaat ttgcctgcat taccggtcga tgcaacgagt 2160
   gatgaggttc gcaagaacct gatggacatg ttcagggatc gccaggcgtt ttctgagcat 2220
   acctggaaaa tgcttctgtc cgtttgccgg tcgtgggcgg catggtgcaa gttgaataac 2280
   cggaaatggt ttcccgcaga acctgaagat gttcgcgatt atcttctata tcttcaggcg 2340
   cgcggtctgg cagtaaaaac tatccagcaa catttgggcc agctaaacat gcttcatcgt 2400
   cggtccgggc tgccacgacc aagtgacagc aatgctgttt cactggttat gcggcggatc 2460
   cgaaaagaaa acgttgatgc cggtgaacgt gcaaaacagg ctctagcgtt cgaacgcact 2520
   gatttcgacc aggttcgttc actcatggaa aatagcgatc gctgccagga tatacgtaat 2580
   ctggcatttc tggggattgc ttataacacc ctgttacgta tagccgaaat tgccaggatc 2640
   agggttaaag atatctcacg tactgacggt gggagaatgt taatccatat tggcagaacg 2700
   aaaacgctgg ttagcaccgc aggtgtagag aaggcactta gcctgggggt aactaaactg 2760
   gtcgagcgat ggatttccgt ctctggtgta gctgatgatc cgaataacta cctgttttgc 2820
   cgggtcagaa aaaatggtgt tgccgcgcca tctgccacca gccagctatc aactcgcgcc 2880
   ctggaaggga tttttgaagc aactcatcga ttgatttacg gcgctaagga tgactctggt 2940
   cagagatacc tggcctggtc tggacacagt gcccgtgtcg gagccgcgcg agatatggcc 3000
   cgcgctggag tttcaatacc ggagatcatg caagctggtg gctggaccaa tgtaaatatt 3060
   gtcatgaact atatccgtac cctggatagt gaaacagggg caatggtgcg cctgctggaa 3120
   gatggcgatt agccattaac gcgtaaatga ttgctataat tatttgatat ttatggtgac 3180
   atatgagaaa ggatttcaac atcgacggaa aatatgtagt gctgtctgta agcactaata 3240
   ttcagtcgcc agccgtcatt gtcactgtaa agctgagcga tagaatgcct gatattgact 3300
   caatatccgt tgcgtttcct gtcaaaagta tgcgtagtgc tgaacatttc gtgatgaatg 3360
   ccaccgagga agaagcacgg cgcggttttg ctaaagtgat gtctgagttt ggcgaactct 3420
   tgggtaaggt tggaattgtc gacctcgagt catgtaatta gttatgtcac gcttacattc 3480
   acgccctccc cccacatccg ctctaaccga aaaggaagga gttagacaac ctgaagtcta 3540
   ggtccctatt tattttttta tagttatgtt agtattaaga acgttattta tatttcaaat 3600
   ttttcttttt tttctgtaca gacgcgtgta cgcatgtaac attatactga aaaccttgct 3660
   tgagaaggtt ttgggacgct cgaaggcttt aatttgcggc cggtacccaa ttcgccctat 3720
   agtgagtcgt attacgcgcg ctcactggcc gtcgttttac aacgtcgtga ctgggaaaac 3780
   cctggcgtta cccaacttaa tcgccttgca gcacatcccc ctttcgccag ctg 3833
<210> 13
   <211> 2715
   <212> DNA
   <213> artificial
<220>
   <223> expression system
<400> 13
   gaattccagc tgtgcttcgg ttacttctaa ggaagtccac acaaatcaag atccgttaga     60
   cgtttcagct tccaaaacag aagaatgtga gaaggcttcc actaaggcta actctcaaca    120
   gacaacaaca cctgcttcat ctgctgttcc agagaaccgt cgacaaccct taatataact    180
   tcgtataatg tatgctatac gaagttatta ggtctagaga tctgtttagc ttgcctcgtc    240
   cccgccgggt cacccggcca gcgacatgga ggcccagaat accctccttg acagtcttga    300
   cgtgcgcagc tcaggggcat gatgtgactg tcgcccgtac atttagccca tacatcccca    360
   tgtataatca tttgcatcca tacattttga tggccgcacg gcgcgaagca aaaattacgg    420
   ctcctcgctg cagacctgcg agcagggaaa cgctcccctc acagacgcgt tgaattgtcc    480
   ccacgccgcg cccctgtaga gaaatataaa aggttaggat ttgccactga ggttcttctt    540
   tcatatactt ccttttaaaa tcttgctagg atacagttct cacatcacat ccgaacataa    600
   acaaccatgg gtaaggaaaa gactcacgtt tcgaggccgc gattaaattc caacatggat    660
   gctgatttat atgggtataa atgggctcgc gataatgtcg ggcaatcagg tgcgacaatc 720
   tatcgattgt atgggaagcc cgatgcgcca gagttgtttc tgaaacatgg caaaggtagc 780
   gttgccaatg atgttacaga tgagatggtc agactaaact ggctgacgga atttatgcct 840
   cttccgacca tcaagcattt tatccgtact cctgatgatg catggttact caccactgcg 900
   atccccggca aaacagcatt ccaggtatta gaagaatatc ctgattcagg tgaaaatatt 960
   gttgatgcgc tggcagtgtt cctgcgccgg ttgcattcga ttcctgtttg taattgtcct 1020
   tttaacagcg atcgcgtatt tcgtctcgct caggcgcaat cacgaatgaa taacggtttg 1080
   gttgatgcga gtgattttga tgacgagcgt aatggctggc ctgttgaaca agtctggaaa 1140
   gaaatgcata agcttttgcc attctcaccg gattcagtcg tcactcatgg tgatttctca 1200
   cttgataacc ttatttttga cgaggggaaa ttaataggtt gtattgatgt tggacgagtc 1260
   ggaatcgcag accgatacca ggatcttgcc atcctatgga actgcctcgg tgagttttct 1320
   ccttcattac agaaacggct ttttcaaaaa tatggtattg ataatcctga tatgaataaa 1380
   ttgcagtttc atttgatgct cgatgagttt ttctaatcag tactgacaat aaaaagattc 1440
   ttgttttcaa gaacttgtca tttgtatagt ttttttatat tgtagttgtt ctattttaat 1500
   caaatgttag cgtgatttat attttttttc gcctcgacat catctgccca gatgcgaagt 1560
   taagtgcgca gaaagtaata tcatgcgtca atcgtatgtg aatgctggtc gctatactgc 1620
   tgtcgattcg atactaacgc cgccatccag tgtcgaaaac gagctcaacc cttaatataa 1680
   cttcgtataa tgtatgctat acgaagttat taggtcaaaa aactagtctt ttaattctgc 1740
   tgtaacccgt acatgcccaa aatagggggc gggttacaca gaatatataa catcgtaggt 1800
   gtctgggtga acagtttatt cctggcatcc actaaatata atggagcccg ctttttaagc 1860
   tggcatccag aaaaaaaaag aatcccagca ccaaaatatt gttttcttca ccaaccatca 1920
   gttcataggt ccattctctt agcgcaacta cagagaacag gggcacaaac aggcaaaaaa 1980
   cgggcacaac ctcaatggag tgatgcaacc tgcctggagt aaatgatgac acaaggcaat 2040
   tcacccacgc atgtatctat ctcatttcct tacaccttct attaccttct gctctctctg 2100
   atttggaaaa agctgaaaaa aaaggttgaa accagttccc tgaaattatt cccctacttg 2160
   actaataagt atataaagac ggtaggtatt gattgtaatt ctgtaaatct atttcttaaa 2220
   cttcttaaat tctactttta tagttagtct tttttttagt tttaaaacac caagaactta 2280
   gtttcgaata aacacacata aacaaacaaa atgagcggcc gcggatccca attgcgactc 2340
   gagtaataaa caggcccctt ttcctttgtc gatctcatgt aattagttat gtcacgctta 2400
   cattcacgcc ctcctcccac atccgctcta accgaaaagg aaggagttag acaacctgaa 2460
   gtctaggtcc ctatttattt tttttaatag ttatgttagt attaagaacg ttatttatat 2520
   ttcaaatttt tctttttttt ctgtacaaac gcgacctgat acaagaactt aacaagaaac 2580
   caattattaa aggcttactt actgatagta gatcaacgat cagtataatt aagtctacaa 2640
   atgaagagaa atttagaaac agattttttg gcacaaaggc aatgagactt agagatgaag 2700
   tatcagctgc tgcag 2715
<210> 14
   <211> 1225
   <212> DNA
   <213> artificial
<220>
   <223> synthetic sequence-expression system
<400> 14
   gaattccagc tgtgcttcgg ttacttctaa ggaagtccac acaaatcaag atccgttaga 60
   cgtttcagct tccaaaacag aagaatgtga gaaggcttcc actaaggcta actctcaaca 120
   gacaacaaca cctgcttcat ctgctgttcc agagaaccgt cgacgatatc gagctcaacc 180
   cttaatataa cttcgtataa tgtatgctat acgaagttat taggtcaaaa aactagtctt 240
   ttaattctgc tgtaacccgt acatgcccaa aatagggggc gggttacaca gaatatataa 300
   catcgtaggt gtctgggtga acagtttatt cctggcatcc actaaatata atggagcccg 360
   ctttttaagc tggcatccag aaaaaaaaag aatcccagca ccaaaatatt gttttcttca 420
   ccaaccatca gttcataggt ccattctctt agcgcaacta cagagaacag gggcacaaac 480
   aggcaaaaaa cgggcacaac ctcaatggag tgatgcaacc tgcctggagt aaatgatgac 540
   acaaggcaat tcacccacgc atgtatctat ctcattttct tacaccttct attaccttct 600
   gctctctctg atttggaaaa agctgaaaaa aaaggttgaa accagttccc tgaaattatt 660
   cccctacttg actaataagt atataaagac ggtaggtatt gattgtaatt ctgtaaatct 720
   atttcttaaa cttcttaaat tctactttta tagttagtct tttttttagt tttaaaacac 780
   caagaactta gtttcgaata aacacacata aacaaacaaa atgagcggcc gcggatccca 840
   attgcgactc gagtaataaa caggcccctt ttcctttgtc gatctcatgt aattagttat 900
   gtcacgctta cattcacgcc ctcctcccac atccgctcta accgaaaagg aaggagttag 960
   acaacctgaa gtctaggtcc ctatttattt tttttaatag ttatgttagt attaagaacg 1020
   ttatttatat ttcaaatttt tctttttttt ctgtacaaac gcgacctgat acaagaactt 1080
   aacaagaaac caattattaa aggcttactt actgatagta gatcaacgat cagtataatt 1140
   aagtctacaa atgaagagaa atttagaaac agattttttg gcacaaaggc aatgagactt 1200
   agagatgaag tatcagctgc tgcag 1225
<210> 15
   <211> 1508
   <212> DNA
   <213> artificial
<220>
   <223> selection insert-expression system
<400> 15
   gtcgacaacc cttaatataa cttcgtataa tgtatgctat acgaagttat taggtctaga     60
   gatctgttta gcttgcctcg tccccgccgg gtcacccggc cagcgacatg gaggcccaga    120
   ataccctcct tgacagtctt gacgtgcgca gctcaggggc atgatgtgac tgtcgcccgt    180
   acatttagcc catacatccc catgtataat catttgcatc catacatttt gatggccgca    240
   cggcgcgaag caaaaattac ggctcctcgc tgcagacctg cgagcaggga aacgctcccc    300
   tcacagacgc gttgaattgt ccccacgccg cgcccctgta gagaaatata aaaggttagg    360
   atttgccact gaggttcttc tttcatatac ttccttttaa aatcttgcta ggatacagtt    420
   ctcacatcac atccgaacat aaacaaccat gggtaaggaa aagactcacg tttcgaggcc    480
   gcgattaaat tccaacatgg atgctgattt atatgggtat aaatgggctc gcgataatgt    540
   cgggcaatca ggtgcgacaa tctatcgatt gtatgggaag cccgatgcgc cagagttgtt    600
   tctgaaacat ggcaaaggta gcgttgccaa tgatgttaca gatgagatgg tcagactaaa    660
   ctggctgacg gaatttatgc ctcttccgac catcaagcat tttatccgta ctcctgatga    720
   tgcatggtta ctcaccactg cgatccccgg caaaacagca ttccaggtat tagaagaata    780
   tcctgattca ggtgaaaata ttgttgatgc gctggcagtg ttcctgcgcc ggttgcattc    840
   gattcctgtt tgtaattgtc cttttaacag cgatcgcgta tttcgtctcg ctcaggcgca    900
   atcacgaatg aataacggtt tggttgatgc gagtgatttt gatgacgagc gtaatggctg    960
   gcctgttgaa caagtctgga aagaaatgca taagcttttg ccattctcac cggattcagt   1020
   cgtcactcat ggtgatttct cacttgataa ccttattttt gacgagggga aattaatagg   1080
   ttgtattgat gttggacgag tcggaatcgc agaccgatac caggatcttg ccatcctatg   1140
   gaactgcctc ggtgagtttt ctccttcatt acagaaacgg ctttttcaaa aatatggtat   1200
   tgataatcct gatatgaata aattgcagtt tcatttgatg ctcgatgagt ttttctaatc   1260
   agtactgaca ataaaaagat tcttgttttc aagaacttgt catttgtata gtttttttat   1320
   attgtagttg ttctatttta atcaaatgtt agcgtgattt atattttttt tcgcctcgac   1380
   atcatctgcc cagatgcgaa gttaagtgcg cagaaagtaa tatcatgcgt caatcgtatg   1440
   tgaatgctgg tcgctatact gctgtcgatt cgatactaac gccgccatcc agtgtcgaaa   1500
   acgagctc                                                             1508